(19) Europäisches Patentamt

European Patent Office

Office européen des brevets

(11) **EP 0 768 896 B1**

(12) **EUROPÄISCHE PATENTSCHRIFT**

(45) Veröffentlichungstag und Bekanntmachung des Hinweises auf die Patenterteilung:
**16.08.2000 Patentblatt 2000/33**

(51) Int Cl.[7]: **A61K 47/14**, A61K 9/20,
A61K 31/565

(21) Anmeldenummer: **95925790.8**

(22) Anmeldetag: **30.06.1995**

(86) Internationale Anmeldenummer:
**PCT/EP95/02536**

(87) Internationale Veröffentlichungsnummer:
**WO 96/01128 (18.01.1996 Gazette 1996/04)**

(54) **NIEDRIG DOSIERTE STEROIDTABLETTEN, DIE GALLUSSÄUREESTER ALS ANTIOXIDANS ENTHALTEN, VERFAHREN ZUR HERSTELLUNG UND DIE VERWENDUNG**

LOW-DOSE STEROID TABLETS CONTAINING GALLIC ACID ESTERS AS ANTIOXIDANT AGENT, PROCESS FOR THE MANUFACTURE OF SAID TABLETS, AND USES OF SAID TABLETS

COMPRIMES DE STEROIDES FAIBLEMENT DOSES, CONTENANT DE L'ESTER D'ACIDE GALLIQUE EN TANT QU'ANTIOXYDANT, LEUR PROCEDE DE FABRICATION ET UTILISATION

(84) Benannte Vertragsstaaten:
**AT BE CH DE DK ES FR GB GR IE IT LI LU MC NL PT SE**

(30) Priorität: **04.07.1994 DE 4424766**
**07.09.1994 DE 4433563**

(43) Veröffentlichungstag der Anmeldung:
**23.04.1997 Patentblatt 1997/17**

(73) Patentinhaber: **SCHERING AKTIENGESELLSCHAFT**
**13353 Berlin (DE)**

(72) Erfinder:
• **BACKENSFELD, Thomas**
**D-10559 Berlin (DE)**
• **LIPP, Ralph**
**14169 Berlin (DE)**
• **KEITEL, Susanne**
**D-10719 Berlin (DE)**

(56) Entgegenhaltungen:
**US-A- 4 436 738**

**Beschreibung**

Die Erfindung betrifft Arzneimittel, die Gallussäureester als Antioxidans in Kombination mit einem steroidalen Wirkstoff enthalten, deren Herstellung und deren Verwendung.

Die vorliegende Erfindung beinhaltet die Herstellung pharmazeutischer Formulierungen, die als Wirkstoff niedrig dosierte Steroidhormone enthalten und durch den Zusatz von Gallussäureester gegen oxidative Abbaureaktionen stabilisiert werden. Die Wirkstoffkonzentrationen in der jeweiligen Darreichungsform, beispielsweise einer Tablette oder einem Dragee betragen üblicherweise 0,1 - 200 µg.

Niedrig dosierte Hormone werden in pharmazeutischen Zubereitungen nach einem wahrscheinlich radikalischen Reaktionsmechanismus oxidativ abgebaut (z. B. zu den entsprechenden 6-Hydroxy-, 6-Keto und delta-6 bzw. delta-9-Verbindungen). Um den Oxidationsprozeß in pharmazeutischen Zubereitungen zu verhindern oder zu verlangsamen und die deklarierte Wirkstoffmenge über die Lagerzeit kontinuierlich gewährleisten zu können, werden häufig Antioxidantien zugesetzt. Die dadurch erzielte erhöhte Stabilität der steroidhaltigen pharmazeutischen Zubereitungen bietet den Vorteil einer längeren Laufzeit ohne besondere Primärpackmittel, wie z.B. Oxyblockbeutel, verwenden zu müssen.

Antioxidantien sind Hilfsstoffe, die in niedrigen Konzentrationen eingesetzt-, imstande sind oxidationsempfindliche Stoffe in Arzneizubereitungen während eines längeren Zeitraums zu schützen oder deren Oxidation zu verzögern. Ganz allgemein handelt es sich um Stabilisatoren oder Oxidationsinhibitoren, die nicht nur in der Pharmazie, sondern auch im Lebensmittel-, Kunststoff- und Kautschuksektor verwendet werden. (H. Sucker, P. Fuchs, P. Speiser in Pharmazeutische Technologie, Thieme Verlag, Stuttgart, 1991; Patent WO 93/17567). In wäßrigen Zubereitungen ist der Wirkungsmechanismus bekannt.

Die Wirkungsweise von Antioxidantien in nicht wäßrigen Systemen wird mit mehrstufigen Radikalkettenmechanismen angenommen, in welche Antioxidantien je nach Typ in bestimmten Stufen eingreifen und dadurch die Kettenmechanismen unterbrechen oder hemmen (K.H. Bauer , K.H. Frömming, C. Führer in Pharmazeutische Technologie, Thieme Verlag, Stuttgart, 1986). In nicht wäßrigen Systemen weden Antioxidantien vorwiegend in halbfesten Zubereitungen wie Salben, Emulsionen und Suspensionen verwendet (Rote Liste 1994, Editio Cantor Aulendorf). So wird beispielsweise durch den Zusatz von Butylhydroxytoluol und/oder Butylhydroxyanisol zu einem corticoidhaltigen Hautpräparat das enthaltene Phosphatid vor Verfärbung geschützt. Die Haltbarkeitsdauer konnte deutlich verlängert werden (US Patent 4,427,670). Die Verwendung von Antioxidantien in unterschiedlichen Kombinationen erhöht die chemische und physikalische Stabilität einer antibiotkahaltigen Augensalbe und der entsprechenden Augentropfen (Drug Dev. and Industrial Pharmacy 19 (1993) S. 2595-2609).

Dahingegen gibt es nur eine geringe Anzahl von festen, oralen Arzneiformen, die Antioxidantien enthalten (Stoffliste, 8. Auflage, Werbe- u. Vertriebsgesellschaft, Eschborn). Beispielsweise wird als Hilfsstoff in Lynestrenol- und Desogestrel-haltige Tablettenpräparate unter anderem DLa-Tocopherol verwendet. (Rote Liste 1994, Editio Cantor, Aulendorf)

In derartigen dispersen System ist jedoch neben der Auswahl des geeigneten Antioxidans die Art und Weise der Einarbeitung des Antioxidans in die pharm. Zubereitung von entscheidender Bedeutung für die angestrebte Stabilitätsverbesserung (Walter Lund in The pharmaceutical codex, 12th edition, The Pharmaceutical Press, 1994, S. 289-292).

Es bestand die Aufgabe, durch den Einsatz von Antioxidantien den massiven oxidativen Abbau des steroidalen Wirkstoffs in den festen oralen Darreichungsformen durch den Einsatz von möglichst niedrigen Konzentrationen des Antioxidans zu verhindern oder zu verlangsamen, so daß Lagerzeiten von bis zu 5 Jahren erreichbar sind.

Ebenso wie bei der Einarbeitung des Wirkstoffs bei der Herstellung von niedrig dosierten Tabletten im Dosisbereich von 0,1 µg bis 200 µg sind auch bei der Einarbeitung des Antioxidans Verdünnungsschritte im Verhältnis 1 : 1000 bis 1: 100.000 durchzuführen. Um die geforderte Dosiergenauigkeit pro einzeldosierte Arzneiform zu gewährleisten, werden nur pharm. Wirkstoffe mit einer bestimmten Korngrößenverteilung eingesetzt. (Pharm. Res. 7 (1990) S. 962 - 966) und / oder Hilfsstoffe mit bestimmten Eigenschaften verwendet (EP 0 503 521 A1: Low dose pharmaceutical preparationes).

Die Aufgabe, den Abbau des steroidalen Wirkstoffs zu verhindern bzw. zu verlangsamen und das Antioxidans möglichst homogen in die festen oralen Darreichungsformen einzuarbeiten wird gemäß der Lehre der Patentansprüche gelöst.

Es wurde gefunden, daß die homogene Verteilung des Antioxidans in der einzeldosierten Arzneiform dadurch erzielt werden kann, daß das Antioxidans in der wässrigen Bindemittellösung aufgelöst wird und somit durch das Versprühen in geeigneten Vorrichtungen, z. B. einem Wirbelschichtgranulator, eingearbeitet wird.

Die Vorteile bei der Verwendung der erfindungsgemäßen Vorgehensweise liegt in der erzielten homogen Verteilung des Antioxidans in der Arzneiform (Tabelle 1). Eine aufwendige Zerkleinerung des Antioxidans mittels Mikronisation kann entfallen. Außerdem

ist der Einsatz von organischen Lösungsmitteln nicht notwendig, was unter Sicherheits- und Umweltgesichtspunkten erstebenswert ist.

Insbesondere wurde gefunden, daß die Einarbeitung des Antioxidans in die Arzneiform über die Granulierflüssigkeit den Vorteil einer effizienteren Stabilisierung bietet, d. h. um eine vergleichbare Stabilitätsverbesserung zu erzielen, wird eine geringere Konzentration des Antioxidans benötigt als wenn das Antioxidans als Feststoff eingearbeitet wird.

Als Antioxidans sind

Alkylester der Gallussäure

z.B. n-Propylgallat (n-Propyl-3,4,5-trihydroxybenzoat) oder Isopropylgallat (iso-Propyl-3,4,5-trihydroxybenzoat) oder Gallussäuremethylester (Methyl-3,4,5-trihydroxybenzoat) oder Gallussäureethylester (Ethyl-3,4,5-trihydroxybenzoat) oder Gallussäurebutylester (n-Butyl-3,4,5-trihydroxybenzoat)

geeignet.

Steroidhormone, die zur Herstellung der erfindungsgemäßen pharmazeutischen Zubereitungen eingesetzt werden können, sind beispielsweise Estrogen wirksame Steroidhormone, wie 1,3,5(10)-Estratrien-3,17β-diol (Estradiol), 1,9-Nor-17α-pregna-1,3,5(10)-trien-20yn-3,17β-diol (Ethinylestradiol), 14α,17α-Ethano-1,3,5(10)-estratrien-3,17β-diol (Cyclodiol), oder 14α,17α-Ethano-1,3,5(10)-estratrien-3,16α, 17β-triol (Cyclotriol); gestagen wirksame Steroidhormone, wie 13-Ethinyl-17β-hydroxy-18,19-dinor-17α-pregna-4,15-dien-20yn-3-on (Gestoden) und dessen Ester oder 17β-Hydroxy-1α-methyl-5α-androsten-3-on (Mesterolon),

Cyproteronacetat,

Estradiolundecyclat,

Estradiolbenzoat,

Estradiolvalerat,

Gestonoroncaprat,

Hydroxyprogesteroncaproat,

Levonorgestrel,

Metenolonacetat,

Norethisteron oder

Norethinsteronacetat,

Norgestrel,

Prasteronenantat,

Testosteron oder

Testosteronpropionat oder

Testosteronenantat,

Das Verfahren zur Herstellung der Arzneimittel, dadurch gekennzeichnet, daß die homogene Verteilung des Antioxydans in der Arzneiform durch Auflösung des Antioxydans in einer wässrigen Bindemittellösung und anschließendes Versprühen in einem Wirbelschichtgranulator erhalten wird.

Es ist naheliegend, daß die erfindungsgemäßen Zubereitungen auch Gemische dieser Wirkstoffe enthalten können.

Die übliche Konzentration an zugesetzter Menge Antioxidans liegt zwischen 0,001 Gew. % und 0,1 Gew. % bezogen auf das Gesamtgewicht der Endformulierung. Diese Konzentration läßt sich auf 5 Gew % steigern; ein üblicher Bereich liegt jedoch bei 0.05 Gew. % Antioxidans.

Die folgenden Beispiele sollen die Erfindung näher erläutern:

**Beispiel 1:**

(10 µg Ethinylestradiol/ Tabl.: 0.01 % Propylgallat)

0,5455 g Ethinylestradiol, 1919,12 g Lactose, 540 g Maisstärke und 360 g modifizierte Stärke werden in einem Wirbelschichtgranulator homogen vermischt. Mit einer wässerigen Polyvinylpyrrolidon 25000 Lösung, die aus 0,3g Propylgallat, 150 g Polyvinylpyrrolidon 25000 und 850g Wasser besteht wird die Pulvermischung im Wirbelschichtgranulator zu einem Granulat verarbeitet. Nach dem Untermischen von 30 g Magnesiumstearat wird die erhaltene Preßmasse zu Tablettenkerne mit einem Gewicht von 55 mg und einem Durchmesser von 5 mm verpreßt. Zusammensetzung einer Tablette:

| Ethinylestradiol | 0,0100 mg |
| Propylgallat | 0,0055 mg |
| Lactose | 35,1845 mg |

(fortgesetzt)

| Maisstärke | 9,9000 mg |
|---|---|
| Modifizierte Stärke | 6,6000 mg |
| Polyvinylpyrrolidon 25000 | 2,7500 mg |
| Magnesiumstearat | 0,5500 mg |
| | $\overline{55,0000\ mg}$ |

**Beispiel 2:**

(20 µg Ethinylestradiol/ Tabl.: 0.05 % Propylgallat)
6 g Ethinylestradiol, 10,542 kg Lactose, 2,97 kg Maisstärke und 1,98 g modifizierte Maisstärke werden in einem Mischer homogen verteilt und in einen Wirbelschichtgranulator überführt. Die Pulvermischung wird mit einer wässerigen Lösung, die aus 8,25g Propylgallat, 825 g Polyvinylpyrrolidon 25000 und 4341g Wasser sowie 4 g Citronensäure besteht im Wirbelschichtgranulator zu einem Granulat verarbeitet. Nach dem Untermischen von 165 g Magnesiumstearat wird die erhaltene Preßmasse zu Tabletten mit einem Gewicht von 55 mg und einem Durchmesser von 5 mm verpreßt.

**Beispiel 3:**

(30 µg Ethinylestradiol/ Tabl.: 0.1 % Methylgallat)
1,6364 g Ethinylestradiol, 1916 g Lactose, 540 g Maisstärke und 360 g modifizierte Stärke werden in einem Wirbelschichtgranulator homogen vermischt. Mit einer wässerigen Polyvinylpyrrolidon 25000 Lösung, die aus 3g Methylgallat, 150 g Polyvinylpyrrolidon 25000 und 847g Wasser besteht wird die Pulvermischung im Wirbelschichtgranulator zu einem Granulat verarbeitet. Nach dem Untermischen von 30 g Magnesiumstearat wird die erhaltene Preßmasse zu Tabletten mit einem Gewicht von 55 mg und einem Durchmesser von 5 mm verpreßt.

**Beispiel 4:**

(25 µg Gestoden/ Tabl.: 0.01 % Propylgallat)
3198 g Lactose, 900 g Maisstärke und 600 g Starch 1500® werden zusammen mit 1,5625 g Gestoden zu einer homogenen Pulvermischung verarbeitet. In einem Mischer wird die Pulvereinwaage mit 1666,5 g einer Lösung, die aus 0,5 g n-Propylgallat, 250 g Polyvinylpyrrolidon 25000 und 1416 g Wasser besteht, zu einem Granulat verarbeitet. Nach dem Egalisieren wird zu dem Granulat 50 g Magnesiumstearat gemischt. Die pressfertige Masse wird auf einer Presse gegeben und zu 80 mg schweren Tabletten mit einem Duchmesser von 6 mm verpreßt.

Tabelle 1

| Gleichförmigkeit des Gehaltes von 0,1 % Propylgallat in Tabletten mit einem Gewicht von 80 mg | | |
|---|---|---|
| | Propylgallat als Feststoff eingearbeitet | Propylgallat über die Bindemittellösung eingearbeitet |
| Mittelwert | 93.2 % | 100.4 % |
| Standardabweichung | 20.8 % | 2.8 % |
| Variationkoeffizient | 22.3 % | 2.8 % |
| Anzahl untersuchter Tabl. | 10 | 10 |

**Patentansprüche**

1.    Verwendung von homogen eingearbeiteten Gallussäureester in einer Konzentration von 0,001 - 5 Gew. % gerechnet zur Gesamtmasse als Antioxydanz zur Herstellung von Arzneimitteln in Form von Tabletten oder Dragees, enthaltend einen steroidalen Wirkstoff in einer Wirkstoffkonzentration von 0,1 - 200 µg.

2.    Arzneimittel nach Anspruch 1, wobei der steroidale Wirkstoff,

      Cylodiol,
      Cyclotriol,

Cyproteronacetat,
Estradiolundecyclat,
Estradiolbenzoat,
Estradiolvalerat,
Ethinylestradiol,
Gestonoroncaprat,
Gestoden,
Hydroxyprogesteroncaproat,
Levonorgestrel,
Mesterolon,
Metenolonacetat,
Norethisteron oder
Norethinsteronacetat,
Norgestrel,
Prasteronenantat,
Testosteron oder
Testosteronpropionat oder
Testosteronenantat,

oder ein Gemisch davon ist.

3. Verfahren zur Herstellung der Arzneimittel nach Anspruch 1, dadurch gekennzeichnet, daß die homogene Verteilung des Antioxydans in der Arzneiform durch Auflösung des Antioxydans in einer wäßrigen Bindemittellösung und anschließendes Versprühen in einem Wirbelschichtgranulator erhalten wird.

## Claims

1. Use of homogeneously incorporated gallic esters in a concentration of 0.001-5% by weight of the total amount as antioxidant for preparing medicaments in the form of tablets or sugar-coated tablets, comprising a steroidal active compound in a concentration of active compound of 0.1-200 µg.

2. Medicament according to Claim 1, where the steroidal active compound is

cyclodiol,
cyclotriol,
cyproterone acetate,
oestradiol undecylate,
oestradiol benzoate,
oestradiol valerate,
ethynyl oestradiol,
gestonorone caproate,
gestodene,
hydroxyprogesterone caproate,
levonorgestrel,
mesterolone,
metenolone acetate,
norethisterone or
norethisterone acetate,
norgestrel,
prasterone enantate,
testosterone or
testosterone propionate or
testosterone enantate,

or a mixture thereof.

3. Process for preparing the medicaments according to Claim 1, characterized in that the homogeneous distribution of the antioxidant in the drug form is obtained by dissolving the antioxidant in an aqueous binder solution, followed by spraying in a fluidized bed granulator.

**Revendications**

1. Utilisation d'esters d'acide gallique incorporés de manière homogène dans une concentration de 0,001-5% en poids, calculée sur base de la masse totale, comme antioxydant pour la préparation de médicaments sous la forme de comprimés ou de dragées, contenant un principe actif stéroïde dans une concentration de principe actif de 0,1-200 μg.

2. Médicament suivant la revendication 1, le principe actif stéroïde étant cyclodiol, cyclotriol, acétate de cyprotérone, undécanoate d'oestradiol, benzoate d'oestradiol, valérate d'oestradiol, l'éthinyloestradiol, caprate de gestonorone, gestoden, caproate d'hydroxyprogestérone, lévonorgestrel, mestérolone, acétate de méténolone, noréthistérone ou l'acétate de norethinstérone, norgestrel, énantate de prastérone, testostérone ou propionate de testostérone ou énantate de testostérone, ou un mélange de ceux-ci.

3. Procédé de préparation des médicaments suivant la revendication 1, caractérisé en ce que la dispersion homogène de l'antioxydant dans la forme médicamenteuse est obtenue par dissolution de l'antioxydant dans une solution aqueuse de liant et par vaporisation ultérieure dans un granulateur à lit fluidisé.